# EUROPEAN PATENT SPECIFICATION

(11) **EP 3 533 438 B1**
(45) Date of publication and mention of the grant of the patent: **21.07.2021**
(21) Application number: 17863748.4
(22) Date of filing: 25.10.2017
(51) Int. Cl.: A61K 8/64, A61K 8/02, A61K 8/34, A61K 8/35, A61Q 19/00, A61K 8/04

(54) **GELATINOUS COMPOSITION**
GELATINÖSE ZUSAMMENSETZUNG
COMPOSITION GÉLATINEUSE

(30) Priority: 28.10.2016 JP 2016211624
(43) Date of publication of application: 04.09.2019
(73) Proprietor: Kaneka Corporation, Osaka-shi, Osaka 530-8288 (JP)
(72) Inventor: TSUJI, Tadao, Settsu-shi Osaka 566-0072 (JP)
(74) Representative: Hoffmann Eitle
(86) International application number: PCT/JP2017/038560
(87) International publication number: WO 2018/079620

(56) References cited:
- EP-B1- 1 082 099
- WO-A1-2016/114340
- JP-A- H10 305 088
- JP-A- 2003 261 424
- JP-A- 2005 247 838
- JP-A- 2006 265 153
- JP-A- 2009 079 030
- JP-A- 2009 137 927
- JP-A- 2015 048 321
- JP-A- 2016 094 398

## Description

### TECHNICAL FIELD

The present invention relates to a highly stable gelatinous composition, and a skin preparation and a cosmetic material comprising the gelatinous composition.

### BACKGROUND ART

Various forms of cosmetics have been developed. There is a gelatinous composition, which is viscous due to an oily component, among such forms. Such an oily viscous gelatinous composition is used for various skin preparations and cosmetic materials, such as cleansing cosmetics and hair cosmetics. As the above-described oily viscous gelatinous composition, a composition containing a polyol and a surfactant has been known. For example, a gelatinous composition containing surfactin, an analogue thereof or a salt thereof as a biosurfactant and a trivalent or higher-valent polyol is known (Patent document 1). Such a gelatinous composition is compatible with skin and exhibits a smooth application texture but has a problem of a stability over time due to a polyol. The use of surfactants derived from a prokaryote such as a Bacillus microbe in external preparations for skin is also known (Patent document 2) .

### PRIOR ART DOCUMENT

### PATENT DOCUMENT

Patent Document 1: JP 2003-176211 A
Patent Document 2: EP 1 082 099 B1

### DISCLOSURE OF THE INVENTION

### PROBLEMS TO BE SOLVED BY THE INVENTION

The objective of the present invention is to provide a gelatinous composition which is compatible with skin and exhibits a smooth application texture but is excellent in a stability over time, and a skin preparation and a cosmetic material comprising the gelatinous composition.

### MEANS FOR SOLVING THE PROBLEMS

The inventors of the present invention intensively studied for solving the above problem. As a result, the present inventors completed the present invention by finding that the stability over time of a gelatinous composition is improved and a gelatinous composition excellent in a feeling of use and a stability over time can be obtained by adding an unsaturated 7-membered cyclic compound in the specific concentration in the gelatinous composition containing a biosurfactant.

Hereinafter, the present invention is described.

### EFFECT OF THE INVENTION

According to the present invention, a gelatinous composition which is compatible with skin and which exhibits a smooth application texture and which is excellent in a stability over time can be provided.

### MODE FOR CARRYING OUT THE INVENTION

The composition of the present invention is gelatinous. The terms "gel" and "gelatinous" in the present invention mean that the composition of the present invention may be soluble or insoluble in a solvent and a viscosity of the composition is 0.01 Pa·s to 2000 Pa·s. When the viscosity is 0.01 Pa·s or more, a problem such as dripping is sufficiently suppressed. When the composition having a viscosity of 2000 Pa·s or less is used as an external preparation or cosmetics for applying to the skin, the composition exhibits an appropriate spreadability. The above viscosity is preferably 0.1 Pa·s or more and 1000 Pa·s or less. The viscosity of the composition may be measured by using a general viscometer. In this disclosure, the phrase "Y to Z" means "Y or more and Z or less", and "Y" and "Z" are included in the present invention range.

The gelatinous composition of the present invention contains a biosurfactant. The biosurfactant contained in the composition of the present invention is a surfactant and exhibits an action of compatibilizing a hydrophilic component such as water and/or a polyol and a lipophilic component such as an oily component.

A biosurfactant is a natural surfactant which is produced by a microorganism. A biosurfactant is characterized by extremely high safety to the environment and the human body, since a biosurfactant is highly biodegradable and has low skin irritation to the human body. The biosurfactant usable in the present invention is exemplified by a lipopeptide compound biosurfactant such as surfactin, arthrofactin and iturin; a glycolipid biosurfactant such as mannosylerythritol lipid, sophorolipid, trehalose lipid and rhamnolipid; a fatty acid biosurfactant such as spiculisporic acid; a polymer biosurfactant such as emulsan; and a salt thereof, and is not restricted thereto.

Among the above examples, a lipopeptide compound biosurfactant such as surfactin, arthrofactin, iturin or a salt thereof is preferred and surfactin or a salt thereof is particularly preferred, since the gelatinous composition effectively becomes stable by using a small amount of the biosurfactant.

The surfactin salt is the compound represented by the general formula (1) or a composition containing the two or more compounds: 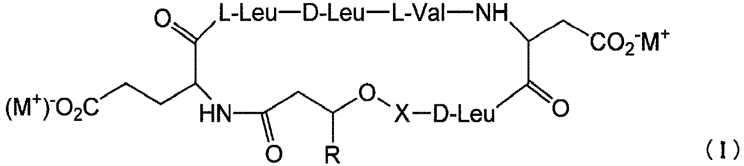 wherein 'X' is an amino acid residue selected from leucine, isoleucine and valine; 'R' is a C₉₋₁₈ alkyl group; 'M⁺' is an alkali metal ion or a quaternary ammonium ion.

The amino acid residue as 'X' may be either in an L-form or a D-form, and the L-form is preferred.

The term "C₉₋₁₈ alkyl group" means a linear or branched monovalent saturated hydrocarbon group having nine or more and eighteen or less carbon atoms. The example thereof includes n-nonyl, 6-methyloctyl, 7-methyloctyl, n-decyl, 8-methylnonyl, n-undecyl, 9-methyldecyl, n-dodecyl, 10-methylundecyl, n-tridecyl, 11-methyldodecyl, n-tetradecyl, n-pentadecyl, n-hexadecyl, n-heptadecyl and n-octadecyl.

The alkali metal ion is not particularly restricted and represents a lithium ion, a sodium ion, a potassium ion or the like.

The example of a substituent of the quaternary ammonium ion includes an organic group exemplified by an alkyl group such as methyl, ethyl, n-propyl, isopropyl, n-butyl and tert-butyl; an aralkyl group such as benzyl, methylbenzyl and phenylethyl; and an aryl group such as phenyl, toluyl and xylyl. The quaternary ammonium ion is exemplified by a tetramethylammonium ion, a tetraethylammonium ion and a pyridinium ion.

Either of one of biosurfactants or not less than two biosurfactants may be used. For example, either of one of the above-described surfactin or salt thereof or not less than two surfactin or salt thereof may be used.

Surfactin or a salt thereof can be isolated from a culture medium prepared by cultivating a microorganism such as a strain belonging to Bacillus subtilis in accordance with a known method. Surfactin or a salt thereof may be a purified product or an unpurified product. Such an unpurified product is exemplified by a culture medium as it is. The product of surfactin or a salt thereof obtained by a chemical synthesis method may be similarly used.

A concentration of a biosurfactant in the gelatinous composition of the present invention is preferably 0.02 wt% to 3 wt%. When the biosurfactant concentration is 0.02 wt% or more, the preservation stability of the gelatinous composition can be sufficiently maintained. When the biosurfactant concentration is 3 wt% or less, a negative effect due to excessive biosurfactant on a feeling of use can be sufficiently inhibited. The concentration is preferably 0.05 wt% to 2 wt%, and more preferably 0.1 wt% to 1.5 wt%. When the gelatinous composition of the present invention contains a component except for the biosurfactant, water and/or polyol, oily component and unsaturated 7-membered cyclic compound, the concentrations of the biosurfactant, water and/or polyol, oily component and unsaturated 7-membered cyclic compound in the gelatinous composition of the present invention mean concentrations in the total of the biosurfactant, water and/or polyol, oily component and unsaturated 7-membered cyclic compound.

The gelatinous composition of the present invention contains water and/or a polyol, in other words, one or more hydrophilic components selected from the group consisting of water and a polyol. Water and a polyol improve the feeling of use of the gelatinous composition according to the present invention as a hydrophilic component and adjust the viscosity.

The polyol means a compound having two or more hydroxy groups. The polyol is not particularly restricted, and it is preferred to use one or more selected from glycerin, diglycerin, 1,2-pentanediol, sorbitol, xylitol and polyethylene glycol. It is also preferred to use a polyol which is in the form of a liquid under an atmospheric temperature and an atmospheric pressure.

A concentration of water and/or a polyol in the gelatinous composition of the present invention is preferably 1 wt% to 50 wt% and more preferably 1.5 wt% to 30 wt%. When the concentration of water and/or a polyol is included in the above-described range, the gelatinous composition having an excellent feeling of use can be produced.

The gelatinous composition of the present invention contains an oily component. Such an oily component appropriately adjusts the viscosity of the gelatinous composition according to the present invention and improves the feeling of use. The oily component usable in the present invention is not particularly restricted as long as the oily component cannot be miscible with water in any proportions, and it is preferred to use one or more selected from a hydrocarbon such as squalane, liquid paraffin, light liquid paraffin, ceresin, polyethylene powder, squalene, microcrystalline wax, petrolatum, liquid isoparaffin, polybutene and mineral oil; a wax such as beeswax, carnauba wax, candelilla wax, jojoba oil, lanolin and spermaceti; a fat and oil such as macadamia nut oil, olive oil, cottonseed oil, soybean oil, avocado oil, rice bran oil, rice oil, rice germ oil, palm kernel oil, castor oil, rose hip oil, evening primrose oil, camellia oil, horse oil, grape seed oil, palm oil, meadow foam oil, shea butter, corn oil, safflower oil and sesame oil; an ester such as ethylhexyl palmitate, isononyl isononanoate, isopropyl myristate, ethyl oleate, glycerol tri-(caprylate/caprate), cetyl 2-ethylhexanoate, glyceryl tri(2-ethylhexanoate), diisopropyl sebacate and cholesteryl hydroxystearate; a long-chain fatty acid such as myristic acid, stearic acid and oleic acid; a silicone oil such as polymethylsiloxane, polymethylphenylsiloxane and an amino-modified silicone; a higher alcohol such as cetanol and oleyl alcohol; an alkyl glyceryl ether such as batyl alcohol and chimyl alcohol.

A concentration of the oily component in the gelatinous composition of the present invention is preferably 50 wt% to 95 wt%. When the concentration of the oily component is included in the above-described range, the gelatinous composition having excellent feeling of use can be obtained. The above-described concentration is preferably 60 wt% to 92 wt%, and more preferably 70 wt% to 90 wt%.

The gelatinous composition of the present invention contains an unsaturated 7-membered cyclic compound. The unsaturated 7-membered cyclic compound has an action to enhance stability of the gelatinous composition according to the present invention by suppressing the separation of the hydrophilic component and the oily component.

The unsaturated 7-membered cyclic compound is hinokitiol (2-hydroxy-4-isopropylcyclohepta-2,4,6-triene-1-one) .

A concentration of the unsaturated 7-membered cyclic compound in the gelatinous composition of the present invention is 0.01 wt% to 3 wt%. When the concentration of the unsaturated 7-membered cyclic compound is included in the above-described range, the stability of the gelatinous composition is improved. The above-described concentration is preferably 0.01 wt% to 1 wt%, and more preferably 0.01 wt% to 0.5 wt%.

An optional component may be contained in the gelatinous composition of the present invention as long as the effect of the present invention can be achieved. Such an optional component is exemplified by a lower alcohol such as ethanol and isopropanol; a surfactant except for the biosurfactant, such as an anionic surfactant, a cationic surfactant, an ampholytic surfactant and a non-ionic surfactant; a thickener, a ultraviolet absorber, an antioxidant, an emollient agent, an emulsifier, a solubilizer, an anti-inflammatory drug, a moisturizer, a preservative, a fungicide, a pH adjuster, a dye, a fragrance and a powder.

The gelatinous composition of the present invention can be produced by mixing the above-described components. In order to make a composition into a gel more efficiently, it is preferred that the biosurfactant and the oily component are mixed in the water and/or polyol and then the unsaturated 7-membered cyclic compound is further mixed into the obtained mixture. The order to add the biosurfactant and the oily component is not particularly restricted, and either of the biosurfactant or the oily component may be first mixed into the water and/or polyol or the biosurfactant and the oily component may be simultaneously added to be mixed. More specifically, for example, the gelatinous composition can be produced by dissolving the biosurfactant in the water and/or polyol, adding the oily component little by little into the stirred solution, and further adding the unsaturated 7-membered cyclic compound little by little thereto. When both of water and the polyol are used in combination, water may be added after the oily component and the unsaturated 7-membered cyclic compound are added. The unsaturated 7-membered cyclic compound may be dissolved in the water and/or polyol and the solution may be added.

The oily component may be added in increments of a predetermined amount or continuously added. The former embodiment is referred to as "split addition" and the latter embodiment is referred to as "continuous addition".

In the case of the split addition, the oily component is added in an amount of 60 mass% or less, preferably 30 mass% or less, and more preferably 10 mass% or less of the already added water and/or polyol per one time, and the mixture is stirred to be homogeneous. By repeating the procedure, a predetermined amount of the oily component is added. In the case of the continuous addition, an addition rate of the oily component is preferably 60 mass% or less of the already added water and/or polyol per one minutes, preferably 30 mass%/min or less, and more preferably 10 mass%/min or less.

In the case where an optional component is added, the optional component may be added by any method. For example, the optional component may be added before the oily component is added, the optional component may be dissolved or dispersed in the oily component and the solution or dispersion may be added, the optional component may be added after the oily component is added, or the optional component may be added while the oily component is added. The water and/or polyol may be first added in a whole amount, or a part of a predetermined amount of the water and/or polyol may be first added and the residual amount may be added later.

For example, the gelatinous composition of the present invention can be preferably used as a skin preparation and a cosmetic material. The gelatinous composition of the present invention can be a skin preparation itself or a cosmetic material itself, and can be mixed with other component to be used as a skin preparation or a cosmetic material. The gelatinous composition of the present invention can be used as, for example, a basic cosmetic material such as cream, lotion, cleansing gel and cleansing cream; a makeup cosmetic material such as foundation, eye shadow, lip color and lip gloss; a hair cosmetic material such as hair cream, styling gel and hair wax; a cleaning product such as shampoo, rinse, hand soap, body soap and cleansing foam.

### EXAMPLES

For example, the gelatinous composition of the present invention is produced as follows; however, the present invention is not restricted to the following Examples.

### Example 1

(A) sodium surfactin 0.8 wt%
(B) glycerin 8 wt%
(C) glycerol tri(caprylate/caprate) 87.69 wt%
(D) hinokitiol 0.01 wt%
(E) ethanol 0.5 wt%
(F) purified water 3 wt%

The components (A) and (C) were added into the component (B) little by little to be dissolved by stirring. Then, the component (D) was dissolved in the component (E), and the solution was added the above solution little by little to be mixed by stirring. The component (F) was further added thereto little by little to be dissolved by stirring to obtain a gelatinous composition.

### Example 2

A gelatinous composition was prepared similarly to Example 1 except that an amount of the component (C) was adjusted to 87.65 wt% and an amount of the component (D) was adjusted to 0.05 wt%.

### Example 3

A gelatinous composition was prepared similarly to Example 1 except that an amount of the component (C) was adjusted to 87.6 wt% and an amount of the component (D) was adjusted to 0.1 wt%.

### Example 4

A gelatinous composition was prepared similarly to Example 1 except that an amount of the component (C) was adjusted to 87.5 wt% and an amount of the component (D) was adjusted to 0.2 wt%.

### Example 5

A gelatinous composition was prepared similarly to Example 1 except that an amount of the component (C) was adjusted to 87.4 wt% and an amount of the component (D) was adjusted to 0.3 wt%.

### Comparative Example 1

A gelatinous composition was prepared similarly to Example 1 except that an amount of the component (C) was adjusted to 87.7 wt% and the component (D) was not used.

### Example 6

(A) sodium surfactin 0.8 wt%
(B) glycerin 18 wt%
(C) squalane 76.69 wt%
(D) hinokitiol 0.01 wt%
(E) 1,2-pentanediol 1.5 wt%
(F) purified water 3 wt%

The components (A) and (C) were added into the component (B) little by little to be dissolved by stirring. Then, the component (D) was dissolved in the component (E), and the solution was added the above solution little by little to be mixed by stirring. The component (F) was further added thereto little by little to be dissolved by stirring to obtain a gelatinous composition.

### Example 7

A gelatinous composition was prepared similarly to Example 6 except that an amount of the component (C) was adjusted to 76.65 wt% and an amount of the component (D) was adjusted to 0.05 wt%.

### Example 8

A gelatinous composition was prepared similarly to Example 6 except that an amount of the component (C) was adjusted to 76.6 wt% and an amount of the component (D) was adjusted to 0.1 wt%.

### Example 9

A gelatinous composition was prepared similarly to Example 6 except that an amount of the component (C) was adjusted to 76.5 wt% and an amount of the component (D) was adjusted to 0.2 wt%.

### Example 10

A gelatinous composition was prepared similarly to Example 6 except that an amount of the component (C) was adjusted to 76.4 wt% and an amount of the component (D) was adjusted to 0.3 wt%.

### Comparative example 2

A gelatinous composition was prepared similarly to Example 6 except that an amount of the component (C) was adjusted to 76.7 wt% and the component (D) was not used.

### Test example 1: Stability evaluation

On the basis of the following standards for stability evaluation, the appearance of the gelatinous compositions of Examples 1 to 5 and Comparative example 1 was evaluated after the compositions were left to stand at 50°C for 4 weeks, and the appearance of the gelatinous compositions of Examples 6 to 10 and Comparative example 2 was evaluated after the compositions were left to stand at 50°C for 8 weeks. The results are shown in Table 1 and Table 2. In Table 1 and Table 2, "SF" represents sodium surfactin.
good: no separation (oily component was not separated.)
bad: separated (oily component was separated.)

**Table 1**

| Component | Example 1 | Example 2 | Example 3 | Example 4 | Example 5 | Comparative example 1 |
|---|---|---|---|---|---|---|
| SF | 0.8 | 0.8 | 0.8 | 0.8 | 0.8 | 0.8 |
| glycerin | 8 | 8 | 8 | 8 | 8 | 8 |
| glycerol tri(caprylate/caprate) | 87.69 | 87.65 | 87.6 | 87.5 | 87.4 | 87.7 |
| hinokitiol | 0.01 | 0.05 | 0.1 | 0.2 | 0.3 | - |
| ethanol | 0.5 | 0.5 | 0.5 | 0.5 | 0.5 | 0.5 |
| purified water | 3 | 3 | 3 | 3 | 3 | 3 |
| total | 100 | 100 | 100 | 100 | 100 | 100 |
| result of stability evaluation (50°C 4W) | good | good | good | good | good | bad |

**Table 2**

| Component | Example 6 | Example 7 | Example 8 | Example 9 | Example 10 | Comparative example 2 |
|---|---|---|---|---|---|---|
| SF | 0.8 | 0. 8 | 0. 8 | 0. 8 | 0.8 | 0. 8 |
| glycerin | 18 | 18 | 18 | 18 | 18 | 18 |
| squalane | 76.69 | 76.65 | 76.6 | 76.5 | 76.4 | 76.7 |
| hinokitiol | 0.01 | 0.05 | 0.1 | 0.2 | 0.3 | - |
| 1,2-pentanediol | 1.5 | 1.5 | 1.5 | 1.5 | 1.5 | 1.5 |
| purified water | 3 | 3 | 3 | 3 | 3 | 3 |
| total | 100 | 100 | 100 | 100 | 100 | 100 |
| result of stability evaluation (50°C 8W) | good | good | good | good | good | bad |

As the result shown in Table 1 and Table 2, the gelatinous compositions of Examples 1 to 10 containing hinokitiol as the unsaturated 7-membered cyclic compound were excellent in preservation stability as the separation of the oily component was not observed in the gelatinous compositions; on the one hand, the gelatinous compositions of Comparative examples 1 and 2 which did not contain hinokitiol had a problem with preservation stability as the oily component was separated in the gelatinous composition.

## Claims

1. A gelatinous composition,
comprising a biosurfactant, water and/or a polyol, an oily component, and an unsaturated 7-membered cyclic compound,
wherein the unsaturated 7-membered cyclic compound is hinokitiol,
wherein a content of the unsaturated 7-membered cyclic compound is 0.01 wt% or more and 3 wt% or less, and
wherein the gelatinous composition is in a gelatinous condition.

2. The gelatinous composition according to claim 1, wherein a concentration of the oily component is 50 wt% or more and 95 wt% or less.

3. The gelatinous composition according to claim 1 or 2, wherein the biosurfactant is one or more selected from the group consisting of surfactin, arthrofactin, iturin and salts thereof.

4. The gelatinous composition according to any one of claims 1 to 3, wherein a concentration of the biosurfactant is 0.02 wt% or more and 3 wt% or less.

5. The gelatinous composition according to any one of claims 1 to 3, wherein a concentration of the biosurfactant is 0.05 wt% or more and 2 wt% or less.

6. The gelatinous composition according to any one of claims 1 to 3, wherein a concentration of the biosurfactant is 0.1 wt% or more and 1.5 wt% or less.

7. The gelatinous composition according to any one of claims 1 to 6, wherein a concentration of the unsaturated 7-membered cyclic compound is 0.01 wt% or more and 1 wt% or less.

8. The gelatinous composition according to any one of claims 1 to 6, wherein a concentration of the unsaturated 7-membered cyclic compound is 0.01 wt% or more and 0.5 wt% or less.

9. The gelatinous composition according to any one of claims 1 to 8, wherein the polyol is one or more selected from the group consisting of glycerin, diglycerin, 1,2-pentanediol, sorbitol, xylitol, polyethyleneglycol and pentanediol.

10. The gelatinous composition according to any one of claims 1 to 8, wherein the polyol is glycerin.

11. The gelatinous composition according to any one of claims 1 to 10, wherein a concentration of the water and/or polyol is 1.5 wt% or more and 30 wt% or less.

12. Use of the gelatinous composition according to any one of claims 1 to 11 in a skin preparation.

13. Use of the gelatinous composition according to any one of claims 1 to 11 in a cosmetic material.

14. A method for producing the gelatinous composition according to any one of claims 1 to 11,
comprising the steps of mixing the biosurfactant and the oily component in the water and/or polyol to obtain a mixture, and further adding the unsaturated 7-membered cyclic compound to the mixture,
wherein the unsaturated 7-membered cyclic compound is hinokitiol, and wherein a content of the unsaturated 7-membered cyclic compound to a total of the biosurfactant, the water and/or polyol, the oily component and the unsaturated 7-membered cyclic compound is 0.01 wt% or more and 3 wt% or less.

## Patentansprüche

1. Gallertartige Zusammensetzung, enthaltend ein Biotensid, Wasser und/oder ein Polyol, eine ölige Komponente und eine ungesättigte, 7-gliedrige, cyclische Verbindung,
worin die ungesättigte, 7-gliedrige, cyclische Verbindung Hinokitiol ist,
worin ein Gehalt der ungesättigten, 7-gliedrigen, cyclischen Verbindung 0,01 Gew.-% oder mehr und 3 Gew.-% oder weniger ist und
worin die gallertartige Zusammensetzung in einem gallertartigen Zustand vorliegt.

2. Gallertartige Zusammensetzung gemäß Anspruch 1, worin eine Konzentration der öligen Komponente 50 Gew.-% oder mehr und 95 Gew.-% oder weniger ist.

3. Gallertartige Zusammensetzung gemäß Anspruch 1 oder 2, worin das Biotensid ein oder mehrere ist, ausgewählt aus der Gruppe, bestehend aus Surfactin, Arthrofactin, Iturin und Salzen davon.

4. Gallertartige Zusammensetzung gemäß einem der Ansprüche 1 bis 3, worin eine Konzentration des Biotensides 0,02 Gew.-% oder mehr und 3 Gew.-% oder weniger ist.

5. Gallertartige Zusammensetzung gemäß einem der Ansprüche 1 bis 3, worin eine Konzentration des Biotensides 0,05 Gew.-% oder mehr und 2 Gew.-% oder weniger ist.

6. Gallertartige Zusammensetzung gemäß einem der Ansprüche 1 bis 3, worin eine Konzentration des Biotensides 0,1 Gew.-% oder mehr und 1,5 Gew.-% oder weniger ist.

7. Gallertartige Zusammensetzung gemäß einem der Ansprüche 1 bis 6, worin eine Konzentration der ungesättigten, 7-gliedrigen, cyclischen Verbindung 0,01 Gew.-% oder mehr und 1 Gew.-% oder weniger ist.

8. Gallertartige Zusammensetzung gemäß einem der Ansprüche 1 bis 6, worin eine Konzentration der ungesättigten, 7-gliedrigen, cyclischen Verbindung 0,01 Gew.-% oder mehr und 5 Gew.-% oder weniger ist.

9. Gallertartige Zusammensetzung gemäß einem der Ansprüche 1 bis 8, worin das Polyol ein oder mehrere ist, ausgewählt aus der Gruppe, bestehend aus Glycerin, Diglycerin, 1,2-Pentandiol, Sorbit, Xylit, Polyethylenglykol und Pentandiol.

10. Gallertartige Zusammensetzung gemäß einem der Ansprüche 1 bis 8, worin das Polyol Glycerin ist.

11. Gallertartige Zusammensetzung gemäß einem der Ansprüche 1 bis 10, worin eine Konzentration von Wasser und/oder Polyol 1,5 Gew.-% oder mehr und 30 Gew.-% oder weniger ist.

12. Verwendung der gallertartigen Zusammensetzung gemäß einem der Ansprüche 1 bis 11 in einem Hautpräparat.

13. Verwendung der gallertartigen Zusammensetzung gemäß einem der Ansprüche 1 bis 11 in einem Kosmetikmaterial.

14. Verfahren zur Erzeugung der gallertartigen Zusammensetzung gemäß einem der Ansprüche 1 bis 11, enthaltend die Schritte zum Mischen des Biotensides und der öligen Komponente in Wasser und/oder Polyol, unter Erhalt einer Mischung, und weitere Zugabe der ungesättigten, 7-gliedrigen, cyclischen Verbindung zu der Mischung,
worin die ungesättigte, 7-gliedrige, cyclische Verbindung Hinokitiol ist und worin ein Gehalt der ungesättigten, 7-gliedrigen, cyclischen Verbindung zu einem Gesamten des Biotensides, Wassers und/oder Polyols, der öligen Komponente und der ungesättigten, 7-gliedrigen, cyclischen Verbindung 0,01 Gew.-% oder mehr und 3 Gew.-% oder weniger ist.

## Revendications

1. Composition gélatineuse,
comprenant un biosurfactant, de l'eau et/ou un polyol, un composant huileux et un composé cyclique non saturé à 7 éléments,
dans laquelle le composé cyclique non saturé à 7 éléments est de l'hinokitiol, dans laquelle une teneur du composé cyclique non saturé à 7 éléments est de 0,01 % en poids ou plus et de 3 % en poids ou moins, et
dans laquelle la composition gélatineuse est dans un état gélatineux.

2. Composition gélatineuse selon la revendication 1, dans laquelle une concentration du composant huileux est de 50% en poids ou plus et de 95% en poids ou moins.

3. Composition gélatineuse selon la revendication 1 ou 2, dans laquelle le biosurfactant est un ou plusieurs sélectionné dans le groupe consistant en de la surfactine, de l'arthrofactine, de l'iturine et des sels de celles-ci.

4. Composition gélatineuse selon l'une quelconque des revendications 1 à 3, dans laquelle une concentration du biosurfactant est de 0,02 % en poids ou plus et de 3 % en poids ou moins.

5. Composition gélatineuse selon l'une quelconque des revendications 1 à 3, dans laquelle une concentration du biosurfactant est de 0,05 % en poids ou plus et de 2 % en poids ou moins.

6. Composition gélatineuse selon l'une quelconque des revendications 1 à 3, dans laquelle une concentration du biosurfactant est de 0,1 % en poids ou plus et de 1,5 % en poids ou moins.

7. Composition gélatineuse selon l'une quelconque des revendications 1 à 6, dans laquelle une concentration du composé cyclique non saturé à 7 éléments est de 0,01 % en poids ou plus et de 1 % en poids ou moins.

8. Composition gélatineuse selon l'une quelconque des revendications 1 à 6, dans laquelle une concentration du composé cyclique non saturé à 7 éléments est de 0,01 % en poids ou plus et de 0,5 % en poids ou moins.

9. Composition gélatineuse selon l'une quelconque des revendications 1 à 8, dans laquelle le polyol est un ou plusieurs sélectionné dans le groupe consistant en de la glycérine, de la diglycérine, du 1,2-pentanédiol, du sorbitol, du xylitol, du polyéthylène glycol et du pentanédiol.

10. Composition gélatineuse selon l'une quelconque des revendications 1 à 8, dans laquelle le polyol est de la glycérine.

11. Composition gélatineuse selon l'une quelconque des revendications 1 à 10, dans laquelle une concentration de l'eau et/ou du polyol est de 1,5 % en poids ou plus et de 30 % en poids ou moins.

12. Utilisation de la composition gélatineuse selon l'une quelconque des revendications 1 à 11 dans une préparation pour la peau.

13. Utilisation de la composition gélatineuse selon l'une quelconque des revendications 1 à 11 dans une matière cosmétique.

14. Procédé de production de la composition gélatineuse selon l'une quelconque des revendications 1 à 11,
comprenant les étapes de mélange du biosurfactant et du composant huileux dans l'eau et/ou le polyol pour obtenir un mélange et en outre d'ajout du composé cyclique non saturé à 7 éléments au mélange,
dans lequel le composé cyclique non saturé à 7 éléments est de l'hinokitiol et dans lequel une teneur du composé cyclique non saturé à 7 éléments par rapport à un total du biosurfactant, de l'eau et/ou du polyol, du composant huileux et du composé cyclique non saturé à 7 éléments est de 0,01 % en poids ou plus et de 3 % en poids ou moins.
